# EUROPEAN PATENT SPECIFICATION

(11) **EP 4 262 669 B1**
(45) Date of publication and mention of the grant of the patent: **21.10.2026**
(21) Application number: 21904744.6
(22) Date of filing: 18.12.2021
(51) Int. Cl.: A61H 3/00, A61F 2/50, A61F 2/60, B25J 9/00, B25J 11/00

(54) **LOAD DISTRIBUTION DEVICE FOR IMPROVING THE MOBILITY OF THE CENTER OF MASS OF A USER DURING COMPLEX MOTIONS**
LASTVERTEILUNGSVORRICHTUNG ZUR VERBESSERUNG DER MOBILITÄT DES MASSENSCHWERPUNKTS EINES BENUTZERS BEI KOMPLEXEN BEWEGUNGEN
DISPOSITIF DE RÉPARTITION DE CHARGE POUR AMÉLIORER LA MOBILITÉ DU CENTRE DE MASSE D'UN UTILISATEUR LORS DE MOUVEMENTS COMPLEXES

(30) Priority: 18.12.2020 US 202063127806 P
(43) Date of publication of application: 25.10.2023
(73) Proprietor: Wistron Corporation, New Taipei City 22181 (TW)
(72) Inventor: ZOSO, Nathaniel, Québec G3A 2J9 (CA); BÉDARD, Stéphane, Québec G3A 2J9 (CA)
(74) Representative: Viering, Jentschura & Partner mbB Patent- und Rechtsanwälte
(86) International application number: PCT/CA2021/051846
(87) International publication number: WO 2022/126284

(56) References cited:
- EP-B1- 1 874 239
- EP-B1- 1 991 180
- EP-B1- 2 621 416
- AU-A1- 2015 201 084
- US-A1- 2013 023 800
- US-A1- 2019 060 157
- US-A1- 2019 254 908
- US-A1- 2019 336 385

## Description

This application claims the benefits of U.S. provisional patent application No. 63/127,806 filed on December 18, 2020.

### TECHNICAL FIELD

The present disclosure relates to a load distribution device for improving the mobility of the center of mass of a user during complex motions.

### BACKGROUND

Lower-body exoskeletons and orthoses provide varying levels of structural and mechanical assistance in specific activities but do so at the cost of reduced joint mobility. Passive devices provide static structural support to the wearer, transferring musculoskeletal stress away from the joints, but lack the ability to provide dynamic assistance. Active solutions provide dynamic assistance in limited situations (e.g., walking gait, sit-to-stand) but do not support complex mobility tasks (e.g., multi-planer motions involving the upper and lower body like swinging a bat, shooting a hockey puck, throwing a ball, or rapid changes in direction and explosive movements of the lower body). Limitations in dynamic assistance devices are due to deficits in their controls (i.e., they are unable to follow the user) or in the range of motion of the supporting structure, limiting the user's ability to optimally control their center of mass in these 3D movements. EP2621416B1 disclosed a load distribution device for transferring musculoskeletal stress from joints to body segments of the lower extremities of a user. The device includes actuation of the hips and knees that follows and assists the user's movement in a complimentary way. The complimentary assistance and load distribution device combine to reduce the loading on the user's joints and increase the user's strength. By assisting the user's hip and/or knees as needed, the device allows the user to achieve improved strength, reduces the metabolic requirements for motion, and increases comfort during physical activity. Certain related technologies and devices are also disclosed in AU2015201084A1, EP1991180B1 and US20130023800A1.

Accordingly, there is a need for a device capable of providing dynamic support to transfer musculoskeletal stress away from the wearer's joints without limiting their function in complex mobility movements, in order to improve the mobility of their center of mass in these movements.

### SUMMARY

The present disclosure provides a load distribution device for improving the mobility of the center of mass of a user during complex motions, comprising:
a pelvic support belt configured to be positioned about a lower trunk of the user;
at least one thigh support element including two or more contact areas configured to be positioned in an agonist-antagonist configuration on a posterior part and an anterior part of a thigh of the user, the at least one thigh support element being rotationally connected to the pelvic support belt;
at least one hip joint actuator providing rotational motion of the at least thigh support element with respect to the pelvic support belt;
at least one shank support element including two or more contact areas configured to be positioned in an agonist-antagonist configuration on a posterior part and an anterior part of a shank of the user, the at least one shank support element being rotationally connected to the at least one thigh support element;
at least one knee joint actuator providing rotational motion of the at least one shank support element with respect to the at least one thigh support element;
a plurality of sensors positioned on the pelvic support belt, the at least one thigh support element, the hip joint actuator and the knee joint actuator, and at least one foot sensor configured to be positioned on a foot of the user, the plurality of sensors providing mechanical and biomechanical signals;
a control unit operatively connected to the plurality of sensors and at least one foot sensor for receiving the mechanical and biomechanical signals, the control unit having stored thereon executable instructions for processing and analyzing the mechanical and biomechanical signals and generating motions set-points of movements of the user; and
a power unit operatively connected to the at least one knee joint actuator, the at least one hip joint actuator and the control unit;
wherein the at least one knee joint actuator and the at least one hip joint actuator both transmit rotational motion to an associated joint of the user via an extension cable and a flexion cable, and transfer musculoskeletal stress from joints to body segments of lower extremities of a user, and therefore improve stability of the joints and a range of motion of the body segments, by generating or dissipating biomechanical energy under directions of the control unit according to a computed level of energy corresponding to a musculoskeletal stress reduction at the joints of the lower extremities of the user necessary to compensate movements of the user, the generated or dissipated biomechanical energy being redistributed onto the lower trunk, the thigh and the shank of the user via the pelvic support belt, the at least one thigh support element and the at least one shank support element, respectively.

The present disclosure also provides a load distribution device as above, comprising two thigh support elements, two shank support elements, two hip joint actuators, two knee joint actuators and two foot sensors.

The present disclosure provides a load distribution device for improving the mobility of the center of mass of a user during complex motions, comprising: a pelvic support belt configured to be positioned about a lower trunk of the user; at least one thigh support element including two or more contact areas configured to be positioned in an agonist-antagonist configuration on a posterior part and an anterior part of a thigh of the user, the at least one thigh support element being rotationally connected to the pelvic support belt; at least one hip joint actuator providing rotational motion of the at least thigh support element with respect to the pelvic support belt; at least one shank support element including two or more contact areas configured to be positioned in an agonist-antagonist configuration on a posterior part and an anterior part of a shank of the user, the at least one shank support element being rotationally connected to the at least one thigh support element; at least one knee joint actuator providing rotational motion of the at least one shank support element with respect to the at least one thigh support element; a plurality of sensors positioned on the pelvic support belt, the at least one thigh support element, the hip joint actuator and the knee joint actuator, and at least one foot sensor configured to be positioned on a foot of the user, the plurality of sensors providing mechanical and biomechanical signals; a control unit operatively connected to the plurality of sensors and at least one foot sensor for receiving the mechanical and biomechanical signals, the control unit having stored thereon executable instructions for processing and analyzing the mechanical and biomechanical signals and generating motions set-points of movements of the user; and a power unit operatively connected to the at least one knee joint actuator, the at least one hip joint actuator and the control unit; wherein the at least one knee joint actuator and the at least one hip joint actuator both transfer musculoskeletal stress from joints to body segments of lower extremities of a user, and therefore improve stability of the joints and a range of motion of the body segments, by generating or dissipating biomechanical energy under directions of the control unit according to a computed level of energy corresponding to a musculoskeletal stress reduction at the joints of the lower extremities of the user necessary to compensate movements of the user, the generated or dissipated biomechanical energy being redistributed onto the lower trunk, the thigh and the shank of the user via the pelvic support belt, the at least one thigh support element and the at least one shank support element, respectively; wherein the load distribution device (10) comprises two thigh support elements (12), two shank support elements (14), two hip joint actuators (22), two knee joint actuators (23) and two foot sensors (45); wherein each of the thigh support elements is rotationally connected to an associated shank support element via a knee pivot aligned with a center of rotation of a knee joint of the user, and wherein each of the knee joint actuators are located remotely from the center of rotation of the knee joint of the user, each of the knee joint actuator both transmitting rotational motion to a corresponding knee pivot via an extension cable and a flexion cable, the extension cable and the flexion cable each include a tension mechanism.

The present disclosure also provides a load distribution device wherein each of the thigh support elements is rotationally connected to the pelvic support belt via a hip pivot aligned with a center of rotation of a hip joint of the user, and wherein each of the hip joint actuators is located remotely from the center of rotation of the hip joint of the user, each of the hip joint actuator transmitting rotational motion to a corresponding hip pivot via an extension cable and a flexion cable.

The present disclosure further provides a load distribution device wherein each of the knee of hip joint actuators may located, for example, medially on a respective side portion of the pelvic support belt, on a lower back portion of the pelvic support belt, on a respective front portion of the thigh of the user, on a respective back portion of the thigh of the user or on a respective portion of the thigh support element between a hip joint of the user and the knee pivot.

The present disclosure further provides a load distribution device further comprising a delocalization mechanism including a deportation structural link having at a first extremity an actuator support element configured to support the knee or hip joint actuator, and a second extremity having a pivot connection element for connecting to the knee or hip pivot. The actuator support element may be configured to removably support the knee or hip joint actuator.

The present disclosure also provides an orthotic device comprising:
a proximal support element including at least one contact area configured to be secured to a proximal body portion of a user and distal support element including at least one contact area configured to be secured to a distal body portion of the user, the proximal support element and the distal support element being rotationally connected via a pivot aligned with a center of rotation of a corresponding joint of the user;
actuators rotationally providing rotational motion of the distal support element with respect to the proximal support element, the actuators being located remotely from the center of rotation of the corresponding joint of the user, wherein the actuators include at least one of knee joint actuators and hip joint actuators, the at least one of the knee joint actuators and hip joint actuators both transmitting rotational motion to the pivot and the associated joint of the user via an extension cable and a flexion cable.

The prosthetic device may further comprise a delocalization mechanism including a deportation structural link having at a first extremity an actuator support element configured to support the actuator, and a second extremity having a pivot connection element for connecting to the pivot. The actuator support element may be configured to removably support the actuator.

### BRIEF DESCRIPTION OF THE FIGURES

Embodiments of the disclosure will be described by way of examples only with reference to the accompanying drawings, in which:
FIGS. 1A, 1 B and 1 C are front, side and back views, respectively, of the load distribution device for improving the mobility of the center of mass of a user during complex motions in accordance with an illustrative embodiment of the present disclosure;
FIGS. 2A and 2B are schematic views of the positioning of the knee joint actuator above the hip joint actuator in accordance with a first and second alternative embodiments of the present disclosure;
FIG. 3 is a schematic view of the positioning of the knee joint actuator above the hip joint actuator and on the lower back of the user in accordance with a third alternative embodiment of the present disclosure;
FIGS. 4A, 4B and 4G are schematic views the positioning of the knee joint actuator above the hip joint actuator and showing cable attachments in a shortened state in accordance with a fourth alternative embodiment of the present disclosure;
FIGS. 5A, 5B and 5C are schematic views the positioning of the knee joint actuator above the hip joint actuator and showing cable attachments in an extended state in accordance with the fourth alternative embodiment of the present disclosure illustrated in FIGS. 4A, 4B and 4C;

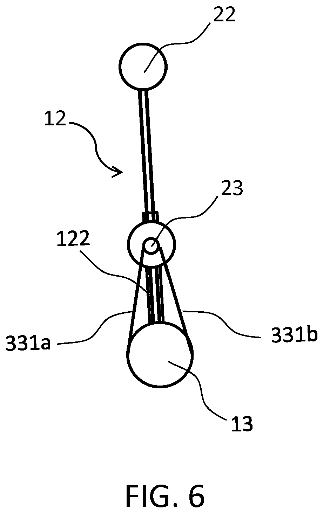

FIG. 6 is a schematic view of the positioning of the knee joint actuator between the hip joint actuator and the knee articulation, in accordance with a fifth alternative embodiment of the present disclosure;
FIGS. 7A, 7B, 4G and 7D are schematic views of various cable attachments in accordance with alliterative embodiments of the present disclosure;
FIGS. 8A, 8B and 8C are a perspective elevated view, a side view and a rear view of an actuator delocalization mechanism in accordance with an illustrative embodiment of the present disclosure;
FIG. 9 is a schematic representation of the load distribution device control system in accordance with the illustrative embodiment of the present disclosure;
FIG. 10 is a flow diagram of the load distribution device control process in accordance with a first illustrative embodiment of the present disclosure;
FIG. 11 is a flow diagram of the load distribution device control process in accordance with a second illustrative embodiment wherein the user's hips and knees are assisted;
FIG. 12 is a flow diagram of the load distribution device control process in accordance with a third illustrative embodiment wherein the user's hips and knees are resisted.

Similar references used in different Figures denote similar components.

### DETAILED DESCRIPTION

Generally stated, the non-limitative illustrative embodiment of the present disclosure provides a load distribution device for improving the mobility of the center of mass of a user during complex motions. The function of the load distribution device is to biomechanically support the pelvic structure of a user during complex motions in order to dynamically improve the mobility the user's center of mass in real-time. This enhances the efficiency of the 3D displacements of the user's center of mass, the stability of related joints, and the range of motion of related body segments. Therefore, the load distribution device improves the overall mobility of the user, which results in advantages such as increased ability to perform desired motions (independently of their level of complexity), metabolic gain in motions, and increased sacro-lumbar, hips and knees stability, which in turn may decrease stress in the dorsal and upper body segments of the user. In order to do so, the load distribution device maintains correct alignment with the user's joints throughout their movements, for example during walking, jogging, running, weight-bearing, squatting, jumping, kneeling, using stairs, participation in sporting activities, and in work-related activities. The device includes actuation of the hips and knees that follows and assists the user's movement in a complimentary way. The complimentary assistance and load distribution device combine to reduce the loading on the user's joints and increase the user's strength. By assisting the user's hip and/or knees as needed, the device allows the user to achieve improved strength, reduces the metabolic requirements for motion, and increases comfort during physical activity. The load distribution device follows the user's limbs through the full joint range of motion and can be used in both passive and active modes.

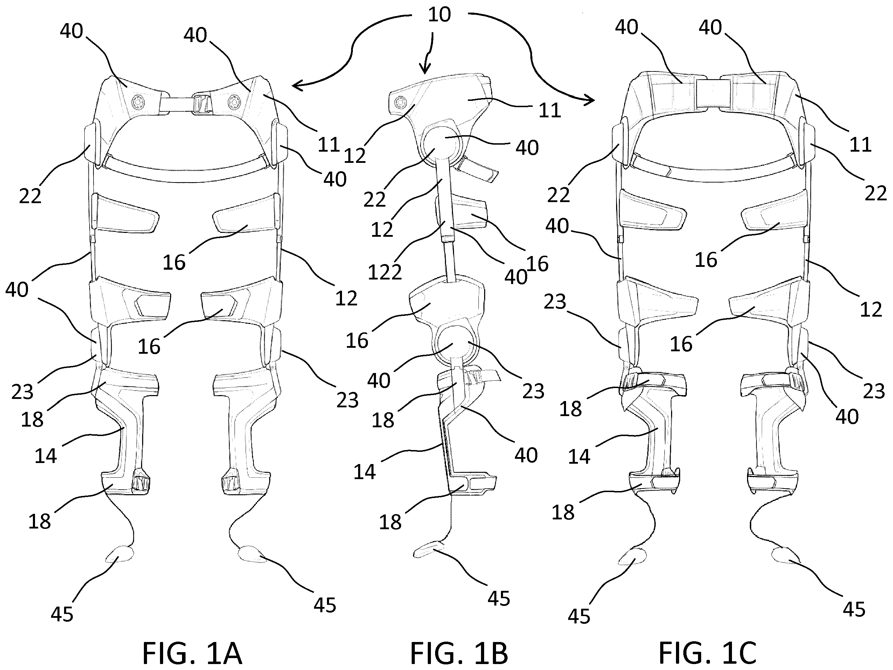

Referring to FIG. 1, the load distribution device for transferring musculoskeletal stress from joints to body segments of the lower extremities of a user 10 comprises a pelvic support belt 11, one or two thigh support elements 12 and one or two shank support elements 14. Advantageously, the pelvic support belt 11 is generally rigid with allowance for some size adjustments, via either extensible portions and/or size adjustment mechanisms.

The pelvic support assembly 11 is configured to be positioned about a lower trunk of the user in an agonist-antagonist configuration and includes a hip joint actuator 22 rotationally connecting the pelvic support belt 11 to the thigh support element 12 and is positioned so as to be aligned with the center of rotation of the hip joint of the user. The hip joint actuator 22 provides active rotational motion at the hip joint of the user. The hip joint actuator 22 may be, for example, an active direct drive rotational actuated mechanism.

The thigh support element 12 include two or more contact areas 16 configured to be positioned in an agonist-antagonist configuration on the posterior and the anterior parts of the thigh of the user.

A knee joint actuator 23 rotationally connect for each of the one or two thigh support elements 12 to the one or two shank support elements 14. The knee joint actuator 23 may be, for example, an active direct drive rotational actuated mechanism.

The shank support element 14 includes two or more contact areas 18 configured to be positioned in an agonist-antagonist configuration on the posterior and the anterior parts of the shank of the user.

A plurality of sensors 40 are positioned on the pelvic support belt 11, either on separate sides or centrally located, the thigh support element 12, the hip joint actuator 22 and the knee joint actuator 23, along with sensors 45 to be positioned at each foot of the user, each of the sensors 40, 45 observing an associated user body segment kinematics in order to provide mechanical and biomechanical information. The sensors 40, 45 may be, for example, inertial and angular sensors.

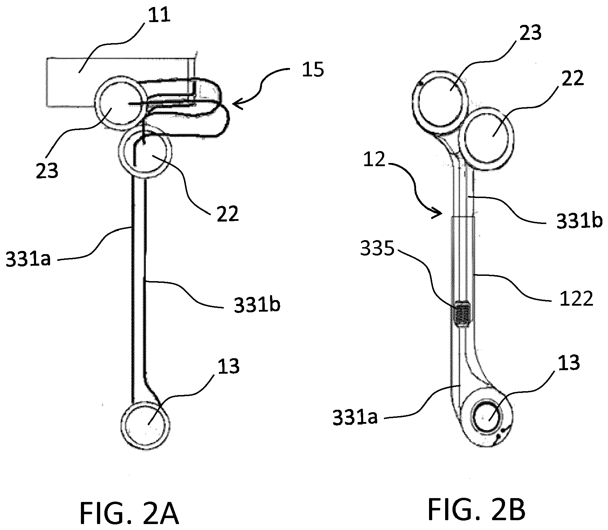

In an alternative embodiment, illustrated in FIG. 2A, the knee joint actuator 23 may be positioned on the pelvic support belt 11 above the hip joint actuator 22, and operatively connected to a knee pivot 13 rotationally connecting the thigh support element 12 to the shank support element 14, in alignment with the center-of-rotation of the knee joint of the user. This allows for a displacement of the weight of the knee joint actuator 23 from the knee joint of the user to the pelvic support belt 11. Rotational motion is transferred from the knee joint actuator 23 to the knee pivot 13 using extension and flexion Bowden cables 331a and 331b, respectively. The Bowden cables 331a, 331b loops 15, which allow for the accommodation of different user heights, are located toward the back of the pelvic support belt 11.

In another alternative embodiment, illustrated in FIG. 2B, the Bowden cables 331a, 331b loop on the knee joint actuator 23. This configuration requires the use of a tensioning mechanism 335 between the knee joint actuator 23 and the knee pivot 13 to manage the tension in the Bowden cables 331a, 331b. This allows for the accommodation of different user heights without having to manage extra cables and avoiding slack so as to obtain good force bandwidth. The tensioning mechanism 335 may be, for example, a Bowden cable tensioner. Screwing the tensioner increases the tension in a corresponding Bowden cable 331a, 331b by elongating its outer casing (sheath).

It should be understood that both the alternative embodiments of FIGS. 2A and 2B are provided with a thigh support element 12 having a length adjustment mechanism 122, for example a slider or a screw mechanism for quick and fine length adjustment.

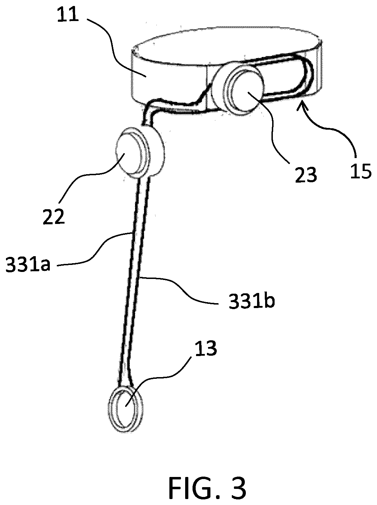

Referring now to FIG. 3, there is shown a further alternative embodiment of the positioning of the knee joint actuator 23, which is positioned on the pelvic support belt 11 above the hip joint actuator 22 and on the lower back of the user. Power is transferred from the knee joint actuator 23 to the knee pivot 13 using extension and flexion Bowden cables 331a and 331b, respectively. The Bowden cables 331a, 331b loops 15, which allow for the accommodation of different user heights, are located toward the back of the pelvic support belt 11.

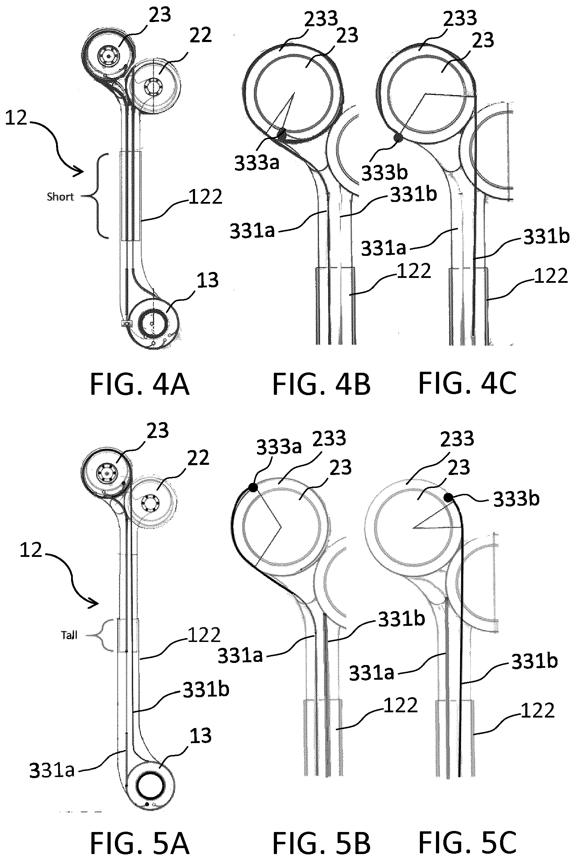

FIGS. 4A, 4B and 4G show a further alternative embodiment of the configuration, in a shortened state, where the knee joint actuator 23 is positioned above the hip joint actuator 22. In this state, the extension and flexion Bowden cables 331a and 331b are connected to the extension 333a and the flexion 333b proximal cable attachments which are positioned around the knee joint actuator 23 such that the extension and flexion Bowden cables 331a and 331b loop around a major portion of the knee joint actuator 23.

FIGS. 5A, 5B and 5C show the alternative embodiment illustrated in FIGS. 4A, 4B and 40, in an extended state. In this state, the extension and flexion Bowden cables 331a and 331b are connected to the extension 333a and the flexion 333b proximal cable attachments which are positioned around the knee joint actuator 23 such that the extension and flexion Bowden cables 331a and 331b loop around a minor portion of the knee joint actuator 23.

The positioning of the extension 333a and the flexion 333b proximal cable attachments may be varied, for example, using adjustment pulleys 233 in order to accommodate various thigh support lengths provided by the length adjustment mechanism 122.

FIG. 5 shows a further still alternative embodiment of the positioning of the knee joint actuator 23, which is positioned between the hip joint actuator 22 and the knee pivot 13.

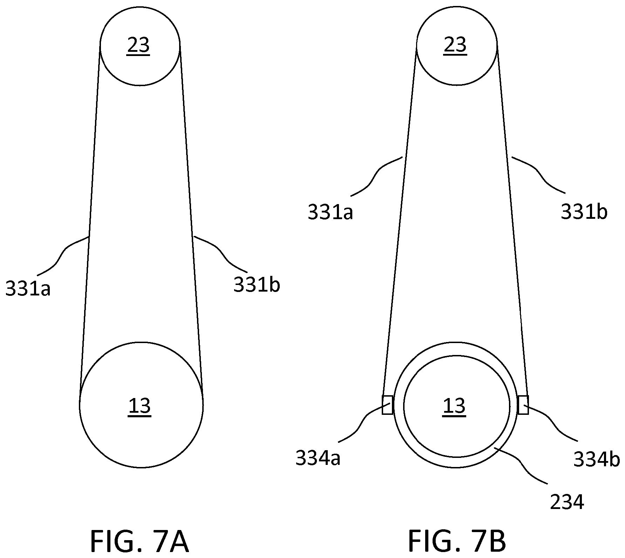

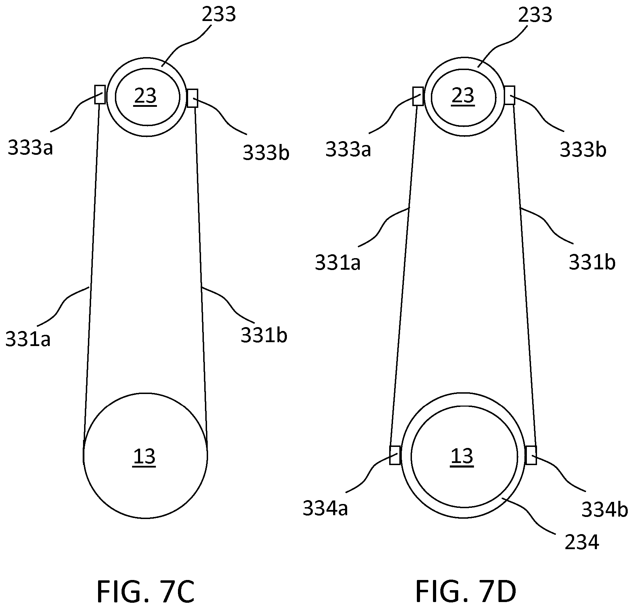

Referring now to FIGS. 7A, 7B, 7C and 7D, there are shown alternative embodiments of the extension 331a and flexion 331b Bowden cables configurations.

In the embodiment of FIG. 7A, the extension 331a and flexion 331b Bowden cables are actually part of a single continuous loop in frictional contact with the stator of the knee joint actuator 23 and knee pivot 13.

In the embodiment of FIG. 7B, the extension 331a and flexion 331b Bowden cables are part of a single cable in frictional contact with the stator of the knee joint actuator 23 and whose two ends are attached to respective extension 334a and flexion 334b distal cable attachments on the knee pivot 13.

In the embodiment of FIG. 7C, the extension 331a and flexion 331b Bowden cables are part of a single cable in frictional contact with the knee pivot 13 and whose two ends are attached to respective extension 333a and flexion 334b proximal cable attachments on the knee joint actuator 23.

In the embodiment of FIG. 7D, the extension 331a and flexion 331b Bowden cables are two separate cables whose ends are attached to respective extension 333a and flexion 333b proximal cable attachments on the knee joint actuator 23 as well as corresponding extension 334a and flexion 334b distal cable attachments on the knee pivot 13.

It is to be understood that the positioning of the proximal 333a, 333b and distal 334a, 334b cable attachments may be varied, for example, using adjustment pulleys 233, 234.

It is to be further understood that in a further alternative embodiment, the hip joint actuator 22 may be displaced and the hip joint provided with a hip pivot similar to the knee pivot 13, with similar alternative embodiments of placement of the hip joint actuator 22 as well as extension 331a and flexion 331b Bowden cables configurations, but for the hip joint actuator 22 and joint instead of the knee joint actuator 23 and joint.

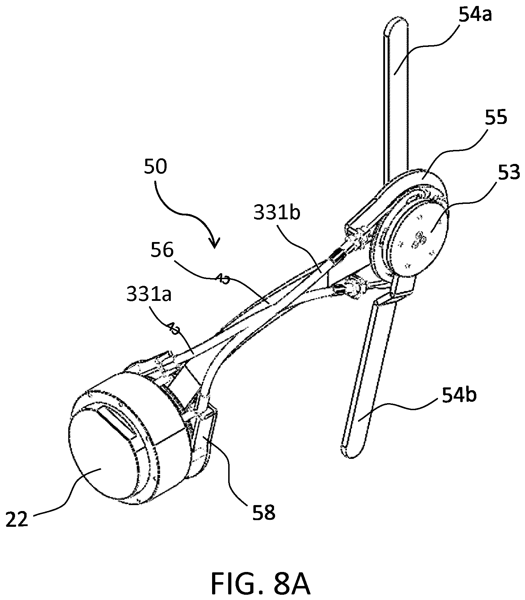

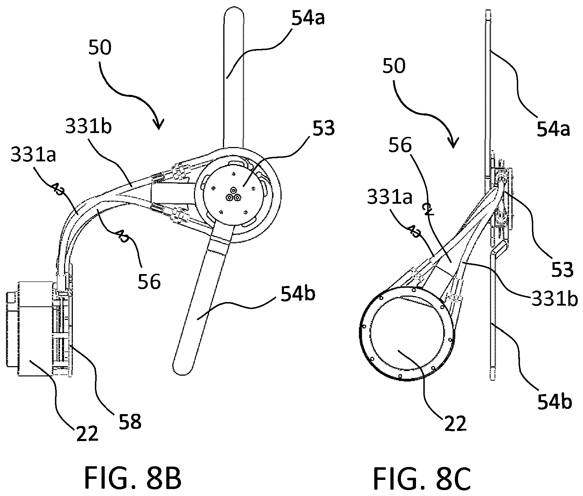

In another alternative embodiment illustrated in FIGS.8A, 8B and 8C, the hip joint actuator 22 may be positioned in a delocalized location with respect to the hip joint of the user using a delocalization mechanism 50. The delocalization mechanism 50 operatively connects the hip joint actuator 22 to a hip pivot 53 in alignment with the center-of-rotation of the hip joint of the user, via a deportation structural link 56. The deportation structural link 56 is provided at one extremity with an actuator support element 58, for supporting the hip joint actuator 22, and at another extremity a hip pivot connection element 55, for connecting to the hip pivot 53. The hip pivot 53 rotationally connects the pelvic support belt 11 to the thigh support element 12 via respective fixation segments 54a and 54b. It is to be understood that in an alternative embodiment the hip joint actuator 22 may be removably secured to the actuator support element 58 for easy removal and replacement of the hip joint actuator 22.

The actuator delocalization mechanism 50 is used to move the center of mass of, or simply displace the volume taken by, the hip joint actuator 22 to a more appropriate location depending on the required use of the load distribution device 10. This allows for a displacement of the weight and volume of the hip joint actuator 22 from the hip joint of the user to another position, for example the front or back of the thigh, or the pelvic support belt 11. Rotational motion is transferred from the hip joint actuator 22 to the hip pivot 53 using extension and flexion Bowden cables 331a and 331b, respectively.

Although disclosed with regard to the hip joint actuator 22, it is to be understood that the delocalization mechanism 50 provides the ability to easily delocalize any actuator, for example the knee joint actuator 23, from the targeted joint while keeping the power directly aligned with the joint, which can be used to improve the aesthetics of the load distribution device 10 or other orthotic device, enhance it functionality and/or adjust its effect on the metabolic cost to a user. In another embodiment, the delocalization mechanism 50 may be used to delocalize an ankle actuator, an elbow actuator or a shoulder actuator.

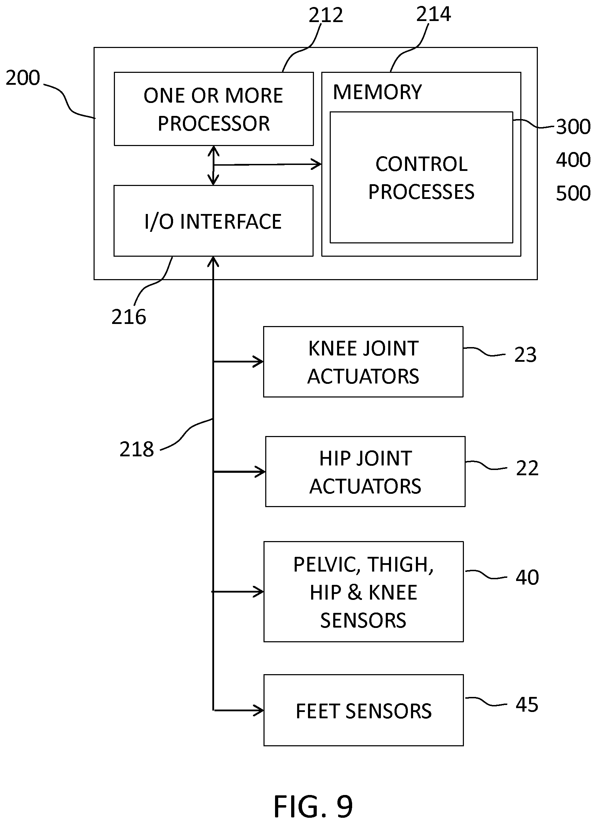

Referring to FIG. 9, control unit 200, which includes load distribution device control processes 300, 400, 500, analyses the mechanical and biomechanical information from the plurality of sensors 40, 45 and provides adaptive tracking and assistance to the user through the load distribution device 10. The load distribution device control system 200 includes one or more processor 212 with an associated memory 214 comprising instructions stored thereon, that when executed on the one or more processor 212, performs the steps of either of the load distribution device control processes 300, 400, 500, which processes will be further described below, and an input/output (I/O) interface 216 for communication with the knee joint actuators 23, the hip joint actuators 22, the pelvic, thigh, hip and knee sensors 40, and the foot sensors 45, through communication link 218, which may be wired, wireless or a combination of both.

A power unit (not shown) provides power to the knee joint actuator 23, the hip joint actuator 22 and the control unit 200.

In use, the knee joint actuator 23 and the hip joint actuator 22 generate and/or dissipate biomechanical energy under directions of the control unit 200 in accordance with user customization and/or mode of operation (e.g., tracking, exercising, etc.), to a computed level of energy corresponding to a musculoskeletal stress reduction, at the joints of the lower extremities of the user, necessary to compensate movements of the user. The generated or dissipated biomechanical energy is then redistributed onto the lower trunk, the thigh and the shank of the user via the pelvic support belt 11, the thigh support element 12 and the shank support element 14, respectively.

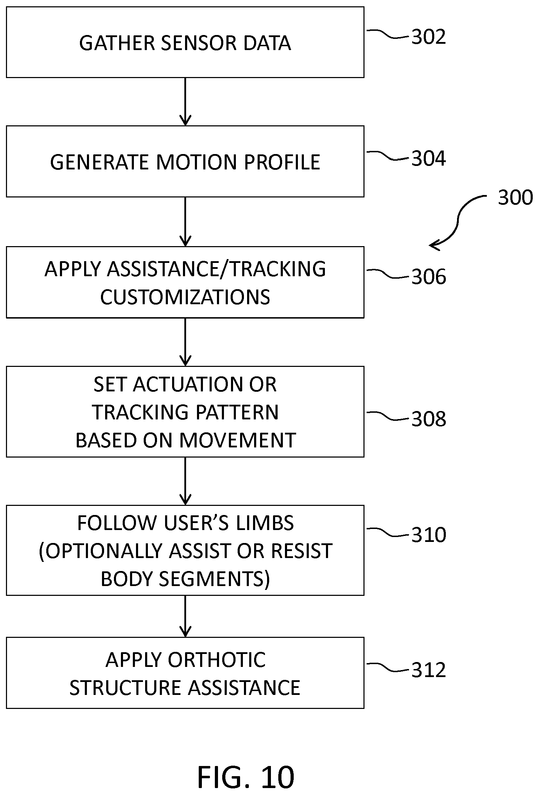

Referring now to FIG. 10, there is shown a flow diagram of the load distribution device control process 300 executed by the one or more processor 212 (see FIG. 9) in accordance with a first illustrative embodiment of the present disclosure. Steps of the process 300 are indicated by blocks 302 to 312.

The process 300 starts at block 302 where process 300 gathers mechanical and biomechanical information of the user acquired from the plurality of sensors 40, 45.

At block 304, the kinematics of the user's body segments are determined using a motion profiler and the gathered mechanical and biomechanical information from block 302.

At block 306, the type of tracking, assistance, and/or resistance offered to the user's limbs is chosen based on the type of application chosen for the system and on the user's customizations of the system settings.

Then, at block 308, the process 200 sets actuation or tracking patterns based on the motion detected by the motion profiler of block and on the user's customizations of block 306, and, at block 310, the process 300 directs the hip 22 and knee 23 actuators to apply either joint actuation to assist or resist the user's motion or provide tracking in a passive mode so as to follow and capture user's limbs kinematics data.

Finally, at block 312, the process 300 controls the load distribution device 10 so that is adapts to the user's natural body motion, allowing free movement unless assistance/resistance of the user's limbs is required.

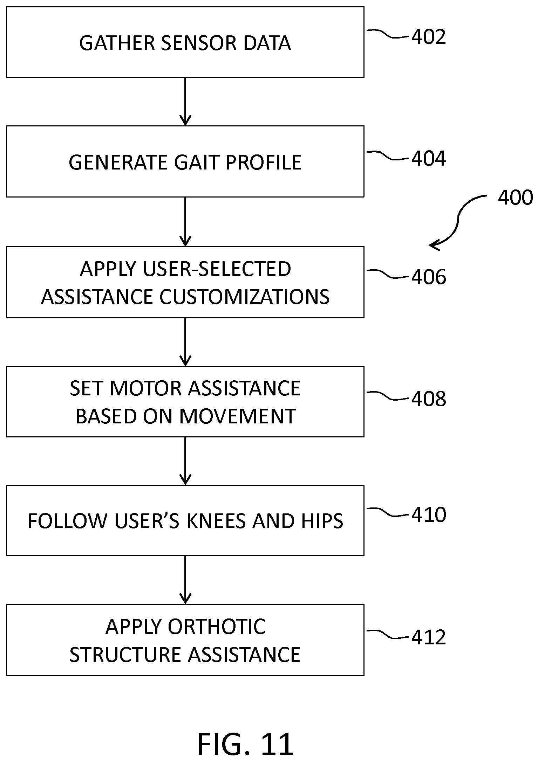

Referring to FIG. 11, there is shown a flow diagram of the load distribution device control process 400 executed by the one or more processor 212 (see FIG. 9) in accordance with a second illustrative embodiment of the present disclosure. Steps of the process 400 are indicated by blocks 402 to 412.

The process 400 starts at block 402 where process 400 gathers mechanical and biomechanical information of the user acquired from the plurality of sensors 40, 45.

At block 404, the kinematics of the user's body segments are determined using a gait profiler and the gathered mechanical and biomechanical information from block 402. The gait profiler may be for example, as disclosed in International Patent Application WO 2018/137016 A1 entitled "Gait Profiler System and Method" filed 25 January 2017.

Then, at block 406, user customization of the magnitude and type of assistance offered by the load distribution device 10 are applied and, at block 408, the process 400 sets the level and timing of assistance offered to the wearer's hips and knees via the hip 22 and knee 23 actuators.

This allows, at block 410, the load distribution device 10 to follow the user's limbs using the detection of user movement pattern and intention provided by the gait profiler.

Finally, at block 412, the process 400 directs the hip 22 and knee 23 actuators to provide supplemental strength to the user to reduce the physiological demands of performing lower body activities. The load distribution device 10 also provides mechanical assistance to reduce the weightbearing burden on the user's joints.

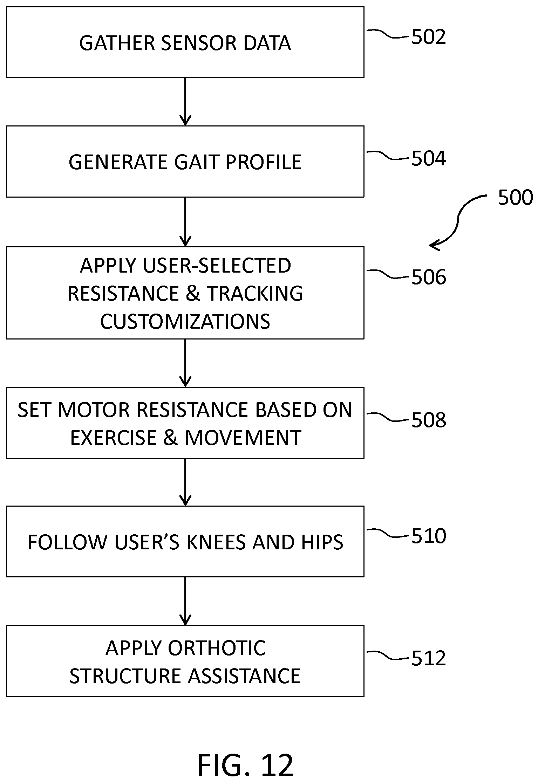

Referring to FIG. 12, there is shown a flow diagram of the load distribution device control process 500 executed by the one or more processor 212 (see FIG. 9) in accordance with a third illustrative embodiment of the present disclosure. Steps of the process 500 are indicated by blocks 502 to 512.

The process 500 starts at block 502 where process 500 gathers mechanical and biomechanical information of the user acquired from the plurality of sensors 40, 45.

At block 504, the kinematics of the user's body segments are determined using a gait profiler and the gathered mechanical and biomechanical information from block 502. The gait profiler may be for example, as disclosed in International Patent Application WO 2018/137016 A1 entitled "Gait Profiler System and Method" filed 25 January 2017.

Then, at block 506, user customization of the magnitude and type of resistance as well as the type of exercise tracking and interactivity (e.g., tracking and signaling rest periods or repetitions) offered by the load distribution device 10 are applied, and, at block 508, the process 500 sets the level and timing of resistance offered to the wearer's hips and/or knees via the hip 22 and knee 23 actuators.

This allows, at block 508, the load distribution device 10 to follow the user's limbs while providing a set level and type of resistance.

Finally, at block 512, the process 500 directs the hip 22 and knee 23 actuators to provide resistance to oppose the motion of the user, allowing him or her to train strength in targeted ranges of motion and types of motion, for example constant joint speed, constant resistance force, targeted power, targeted pace, etc.

The selection of the type of tracking, assistance, and/or resistance offered to the user's limbs, magnitude and type of assistance provided, as well as the type of exercise tracking and interactivity may be performed using mechanical inputs (e.g., buttons) and/or digital inputs the located on the load distribution device 10 and/or via a software application on a peripheral device such as a remote control or a. smart phone.

It is to be understood that the various embodiments of the load distribution device disclosed therein may be provided with or without length adjustment capabilities and may be designed for specific user physiologies.

It is to be further understood that the load distribution device for transferring musculoskeletal stress from joints to body segments of the lower extremities of a user may be provided to one or both of the user's lower body extremities.

Although the present disclosure has been described by way of particular non-limiting illustrative embodiments and examples thereof, it should be noted that it will be apparent to persons skilled in the art that modifications may be applied to the present particular embodiment without departing from the scope of the present disclosure.

## Claims

1. A load distribution device (10) for improving the mobility of the center of mass of a user during complex motions, comprising:
a pelvic support belt (11) configured to be positioned about a lower trunk of the user;
at least one thigh support element (12) including two or more contact areas (18) configured to be positioned in an agonist-antagonist configuration on a posterior part and an anterior part of a thigh of the user, the at least one thigh support element (12) being rotationally connected to the pelvic support belt (11);
at least one hip joint actuator (22) providing rotational motion of the at least thigh support element (12) with respect to the pelvic support belt (11);
at least one shank support element (14) including two or more contact areas (18) configured to be positioned in an agonist-antagonist configuration on a posterior part and an anterior part of a shank of the user, the at least one shank support element (14) being rotationally connected to the at least one thigh support element (12);
at least one knee joint actuator (23) providing rotational motion of the at least one shank support element (14) with respect to the at least one thigh support element (12); a plurality of sensors (40) positioned on the pelvic support belt (11), the at least one thigh support element (12), the hip joint actuator (22) and the knee joint actuator (23), and at least one foot sensor (45) configured to be positioned on a foot of the user, the plurality of sensors (40, 45) providing mechanical and biomechanical signals;
a control unit (200) operatively connected to the plurality of sensors (40) and at least one foot sensor (45) for receiving the mechanical and biomechanical signals, the control unit (200) having stored thereon executable instructions for processing and analyzing the mechanical and biomechanical signals and generating motions set-points of movements of the user; and
a power unit operatively connected to the at least one knee joint actuator (23), the at least one hip joint actuator (22) and the control unit (200);
**characterized in that**
the at least one knee joint actuator (23) and the at least one hip joint actuator (22) both transmit rotational motion to an associated joint of the user via an extension cable (331a) and a flexion cable (331b), and transfer musculoskeletal stress from joints to body segments of lower extremities of a user, and therefore improve stability of the joints and a range of motion of the body segments, by generating or dissipating biomechanical energy under directions of the control unit (200) according to a computed level of energy corresponding to a musculoskeletal stress reduction at the joints of the lower extremities of the user necessary to compensate movements of the user, the generated or dissipated biomechanical energy being redistributed onto the lower trunk, the thigh and the shank of the user via the pelvic support belt (11), the at least one thigh support element (12) and the at least one shank support element (14), respectively.

2. The load distribution device (10) of claim 1, comprising two thigh support elements (12), two shank support elements (14), two hip joint actuators (22), two knee joint actuators (23) and two foot sensors (45).

3. The load distribution device (10) of claim 1,
wherein the at least one knee joint actuator (23) and the at least one hip joint actuator (22) both transfer musculoskeletal stress from joints to body segments of lower extremities of a user, and therefore improve stability of the joints and a range of motion of the body segments, by generating or dissipating biomechanical energy under directions of the control unit (200) according to a computed level of energy corresponding to a musculoskeletal stress reduction at the joints of the lower extremities of the user necessary to compensate movements of the user, the generated or dissipated biomechanical energy being redistributed onto the lower trunk, the thigh and the shank of the user via the pelvic support belt (11), the at least one thigh support element (12) and the at least one shank support element (14), respectively;
wherein the load distribution device (10) comprises two thigh support elements (12), two shank support elements (14), two hip joint actuators (22), two knee joint actuators (23) and two foot sensors (45);
wherein each of the thigh support elements (12) is rotationally connected to at least one of a shank support element (14) via a knee pivot (13) aligned with a center of rotation of a knee joint of the user and the pelvic support belt (11) via a hip pivot (53) aligned with a center of rotation of a hip joint of the user, and wherein at least one of the knee joint actuators (23) and the hip joint actuators (22) is located remotely from the center of rotation of an associated joint of the user, the at least one of the knee joint actuators (23) and the hip joint actuators (22) both transmitting rotational motion to the associated joint of the user via an extension cable (331a) and a flexion cable (331b), the extension cable (331a) and the flexion cable (331b) each include a tension mechanism.

4. The load distribution device (10) of claim 3, wherein each of the knee joint actuators (23) and each of the hip joint actuators (22) are located in a location selected from the group consisting of medially on a respective side portion of the pelvic support belt (11), on a lower back portion of the pelvic support belt (11), on a respective front portion of the thigh of the user, on a respective back portion of the thigh of the user and on a respective portion of the thigh support element (12) between a hip joint of the user and a knee joint of the user.

5. The load distribution device (10) of either one of claims 3 or 4, wherein the thigh support elements (12) include a respective length adjustment mechanism (122).

6. The load distribution device (10) of claim 5, wherein the length adjustment mechanism (122) is selected from a group consisting of a slider mechanism and a screw mechanism.

7. The load distribution device (10) of either one of claims 3 or 4, further comprising a delocalization mechanism (50) including a deportation structural link (56) having at a first extremity an actuator support element (58) configured to support one of the knee joint actuator (23) and the hip joint actuator (22), and a second extremity having a pivot connection element (55) for connecting a corresponding one of the knee pivot (13) and the hip pivot (53).

8. The load distribution device (10) of claim 7, wherein the actuator support element (58) is configured to removably support the one of the knee joint actuator (23) and the hip joint actuator (22).

9. The load distribution device (10) of any one of claims 3 to 8, wherein the extension cable 331a and the flexion cable 331b are formed by a single cable.

10. An orthotic device for a load distribution device according to claim 1, comprising:
a proximal support element including at least one contact area (18) configured to be secured to a proximal body portion of a user and distal support element including at least one contact area (18) configured to be secured to a distal body portion of the user, the proximal support element and the distal support element being rotationally connected via a pivot (13, 53) aligned with a center of rotation of a corresponding joint of the user;
actuators (22, 23) rotationally providing rotational motion of the distal support element with respect to the proximal support element, the actuators (22, 23) being located remotely from the center of rotation of the corresponding joint of the user, wherein the actuators (22, 23) include at least one of knee joint actuators (23) and hip joint actuators (22), the at least one of the knee joint actuators (23) and hip joint actuators (22) both transmitting rotational motion to the pivot and the associated joint of the user via an extension cable (331a) and a flexion cable (331b).

11. The orthotic device of claim 10, further comprising a delocalization mechanism (50) including a deportation structural link (56) having at a first extremity an actuator support element (58) configured to support the actuator (22, 23), and a second extremity having a pivot connection element (55) for connecting to the pivot (13, 53).

12. The orthotic device of claim 11, wherein the actuator support element (58) is configured to removably support the actuator (22, 23).

13. The orthotic device of either one of claims 11or 12, wherein the pivot is selected from a group consisting of a knee pivot (13), a hip pivot (53), an ankle pivot, an elbow pivot and a shoulder pivot.

14. The orthotic device of any one of claims 10 to 13, wherein the extension cable 331a and the flexion cable 331b are formed by a single cable.

## Patentansprüche

1. Lastverteilungsvorrichtung (10) zur Verbesserung der Beweglichkeit des Schwerpunkts eines Benutzers bei komplexen Bewegungen, aufweisend:
einen Beckenstützgurt (11), eingerichtet, um den unteren Rumpf des Benutzers positioniert zu werden;
mindestens ein Oberschenkelstützelement (12), eingerichtet, in einer Agonist-Antagonist-Konfiguration an einem hinteren und einem vorderen Teil eines Oberschenkels des Benutzers positioniert zu werden, wobei das mindestens eine Oberschenkelstützelement (12) drehbar mit dem Beckenstützgurt (11) verbunden ist und zwei oder mehr Kontaktflächen (18) aufweist;
mindestens einen Hüftgelenk-Aktuator (22), der eine Drehbewegung des mindestens einen Oberschenkelstützelements (12) relativ zum Beckenstützgurt (11) ermöglicht;
mindestens ein Schienbeinstützelement (14), eingerichtet, in einer Agonist-Antagonist-Konfiguration an einem hinteren und einem vorderen Teil des Schienbeins des Benutzers positioniert zu werden, wobei das mindestens eine Schienbeinstützelement (14) drehbar mit dem mindestens einen Oberschenkelstützelement (12) verbunden ist und zwei oder mehr Kontaktflächen (18) aufweist;
mindestens ein Kniegelenk-Aktuator (23), der eine Drehbewegung des mindestens einen Schienbeinstützelements (14) relativ zu dem mindestens einen Oberschenkelstützelement (12) ermöglicht;
eine Vielzahl von Sensoren (40), angeordnet an dem Beckenstützgurt (11), dem mindestens einen Oberschenkelstützelement (12), dem Hüftgelenk-Aktuator (22) und dem Kniegelenk-Aktuator (23), sowie mindestens einem Fußsensor (45), wobei der Fußsensor (45) eingerichtet ist, an einem Fuß des Benutzers positioniert zu werden, wobei die Vielzahl von Sensoren (40, 45) mechanische und biomechanische Signale bereitstellen;
eine Steuereinheit (200), funktionsmäßig verbunden mit der Vielzahl von Sensoren (40) und dem mindestens einem Fußsensor (45), um die mechanischen und biomechanischen Signale zu empfangen, wobei in der Steuereinheit (200) ausführbare Befehle zur Verarbeitung und Analyse der mechanischen und biomechanischen Signale sowie zur Erzeugung von Bewegungssollwerten für die Bewegungen des Benutzers gespeichert sind; und
eine Antriebseinheit, funktionsmäßig verbunden mit dem mindestens einen Kniegelenk-Aktuator (23), dem mindestens einen Hüftgelenk-Aktuator (22) und der Steuereinheit (200);
**dadurch gekennzeichnet, dass**
der mindestens eine Kniegelenk-Aktuator (23) und der mindestens eine Hüftgelenk-Aktuator (22) beide eine Drehbewegung auf ein zugehöriges Gelenk des Benutzers über ein Streckkabel (331a) und ein Beugekabel (331b) übertragen, und muskuloskelettale Belastungen von den Gelenken auf Körpersegmente der unteren Extremitäten eines Benutzers übertragen, und dadurch die Stabilität der Gelenke und den Bewegungsumfang der Körpersegmente verbessern, indem sie unter der Steuerung der Steuereinheit (200) biomechanische Energie erzeugen oder ableiten, entsprechend einem berechneten Energieniveau, korrespondierend zu einer Reduzierung der muskuloskelettalen Belastung an den Gelenken der unteren Extremitäten des Benutzers, die zum Ausgleich der Bewegungen des Benutzers erforderlich ist, wobei die erzeugte oder abgeleitete biomechanische Energie jeweils über den Beckenstützgurt (11), das mindestens eine Oberschenkelstützelement (12) und das mindestens eine Unterschenkelstützelement (14) auf den unteren Rumpf, den Oberschenkel und den Unterschenkel des Benutzers umverteilt wird.

2. Lastverteilungsvorrichtung (10) nach Anspruch 1, aufweisend zwei Oberschenkelstützelemente (12), zwei Unterschenkelstützelemente (14), zwei Hüftgelenk-Aktuatoren (22), zwei Kniegelenk-Aktuatoren (23) und zwei Fußsensoren (45).

3. Lastverteilungsvorrichtung (10) nach Anspruch 1,
wobei der mindestens eine Kniegelenk-Aktuator (23) und der mindestens eine Hüftgelenk-Aktuator (22) muskuloskelettale Belastungen von den Gelenken auf Körpersegmente der unteren Extremitäten eines Benutzers übertragen und somit die Stabilität der Gelenke und den Bewegungsumfang der Körpersegmente verbessern, durch Erzeugung oder Abgabe biomechanischer Energie gemäß Anweisungen der Steuereinheit (200) entsprechend einem berechneten Energieniveau, korrespondierend zu einer Reduzierung der muskuloskelettalen Belastung an den Gelenken der unteren Extremitäten des Benutzers, die zum Ausgleich von Bewegungen des Benutzers erforderlich ist, wobei die erzeugte oder abgeleitete biomechanische Energie jeweils über den Beckenstützgurt (11), das mindestens eine Oberschenkelstützelement (12) und das mindestens eine Unterschenkelstützelement (14) auf den unteren Rumpf, den Oberschenkel und den Unterschenkel des Benutzers umverteilt wird;
wobei die Lastverteilungsvorrichtung (10) zwei Oberschenkelstützelemente (12), zwei Unterschenkelstützelemente (14), zwei Hüftgelenk-Aktuatoren (22), zwei Kniegelenk-Aktuatoren (23) und zwei Fußsensoren (45) umfasst;
wobei jedes der Oberschenkelstützelemente (12) drehbar mit mindestens einem der Unterschenkelstützelemente (14) über einen Kniedrehpunkt (13), der auf den Drehpunkt eines Kniegelenks des Benutzers ausgerichtet ist, und mit dem Beckenstützgurt (11) über einen Hüftdrehpunkt (53), der auf den Drehpunkt eines Hüftgelenks des Benutzers ausgerichtet ist, verbunden ist, und wobei mindestens einer der Kniegelenk-Aktuatoren (23) und der Hüftgelenk-Aktuatoren (22) entfernt vom Drehzentrum eines zugehörigen Gelenks des Benutzers angeordnet ist, wobei der mindestens eine der Kniegelenk-Aktuatoren (23) und die Hüftgelenk-Aktuatoren (22) Drehbewegung über ein Streckkabel (331a) und ein Beugekabel (331b) auf das zugehörige Gelenk des Benutzers übertragen, wobei das Streckkabel (331a) und das Beugekabel (331b) jeweils einen Spannmechanismus umfassen.

4. Lastverteilungsvorrichtung (10) nach Anspruch 3, wobei jeder der Kniegelenk-Aktuatoren (23) und jeder der Hüftgelenk-Aktuatoren (22) an einer Stelle angeordnet ist, die aus der Gruppe bestehend aus: mittig an einem jeweiligen Seitenabschnitt des Beckenstützgurts (11), an einem unteren Rückenabschnitt des Beckenstützgurts (11), an einem jeweiligen vorderen Abschnitt des Oberschenkels des Benutzers, an einem jeweiligen hinteren Abschnitt des Oberschenkels des Benutzers und an einem jeweiligen Abschnitt des Oberschenkelstützelements (12) zwischen einem Hüftgelenk des Benutzers und einem Kniegelenk des Benutzers, ausgewählt ist.

5. Lastverteilungsvorrichtung (10) nach einem der Ansprüche 3 oder 4, wobei die Oberschenkelstützelemente (12) jeweils einen Längenverstellmechanismus (122) aufweisen.

6. Lastverteilungsvorrichtung (10) nach Anspruch 5, wobei der Längenverstellmechanismus (122) aus einer Gruppe bestehend aus einem Schiebemechanismus und einem Schraubmechanismus ausgewählt ist.

7. Lastverteilungsvorrichtung (10) nach einem der Ansprüche 3 oder 4, ferner aufweisend:
einen Delokalisierungsmechanismus (50), aufweisend ein Ablenkungsverbindungsglied (56), das an einer ersten Extremität ein Aktuator-Stützelement (58) aufweist, das eingerichtet ist, den Kniegelenk-Aktuator (23) oder den Hüftgelenk-Aktuator (22) zu stützen, und an einer zweiten Extremität ein Schwenkverbindungselement (55) zur Verbindung mit dem Kniegdrehpunkt (13) bzw. dem Hüftdrehpunkt (53) aufweist.

8. Lastverteilungsvorrichtung (10) nach Anspruch 7, wobei das Aktuator-Stützelement (58) eingerichtet ist, den Kniegelenk-Aktuator (23) oder den Hüftgelenk-Aktuator (22) lösbar zu stützen.

9. Lastverteilungsvorrichtung (10) nach einem der Ansprüche 3 bis 8, wobei das Streckkabel (331a) und das Beugekabel (331b) aus einem einzigen Kabel bestehen.

10. Orthese für eine Lastverteilungsvorrichtung (10) nach Anspruch 1, aufweisend:
ein Proximal-Stützelement, eingerichtet an einem proximalen Körperabschnitt eines Benutzers befestigt zu werden, wobei das Proximal-Stützelement mindestens einem Kontaktbereich (18) aufweist, und
ein Distal-Stützelement, eingerichtet an einem distalen Körperabschnitt des Benutzers befestigt zu werden, wobei das Distal-Stützelement mindestens einem Kontaktbereich (18) aufweist,
wobei das Proximal-Stützelement und das Distal-Stützelement über einen Drehpunkt (13, 53) drehbar miteinander verbunden sind, der auf den Drehpunkt eines entsprechenden Gelenks des Benutzers ausgerichtet ist;
Aktuatoren (22, 23), die eine Drehbewegung des Distal-Stützelements relativ zum Proximal-Stützelement ermöglichen, wobei die Aktuatoren (22, 23) entfernt vom Drehpunkt des entsprechenden Gelenks des Benutzers angeordnet sind, wobei die Aktuatoren (22, 23) mindestens einen der Kniegelenk-Aktuatoren (23) und Hüftgelenk-Aktuatoren (22) umfassen, wobei der mindestens eine der Kniegelenk-Aktuatoren (23) und Hüftgelenk-Aktuatoren (22) die Drehbewegung über ein Streckkabel (331a) und ein Beugekabel (331b) sowohl auf den Drehpunkt als auch auf das zugehörige Gelenk des Benutzers überträgt.

11. Orthese nach Anspruch 10, ferner aufweisend:
einen Delokalisierungsmechanismus (50), aufweisend ein Ablenkungsverbindungsglied (56), das an einer ersten Extremität ein Aktuator-Stützelement (58) aufweist, das eingerichtet ist, den Kniegelenk-Aktuator (23) oder den Hüftgelenk-Aktuator (22) zu stützen, und an einer zweiten Extremität ein Schwenkverbindungselement (55) zur Verbindung mit dem Drehpunkt (13, 53) aufweist.

12. Orthese nach Anspruch 11, wobei das Aktuator-Stützelement (58) eingerichtet ist, den Aktuator (22, 23) lösbar zu stützen.

13. Orthese nach einem der Ansprüche 11 oder 12, wobei der Drehpunkt aus einer Gruppe ausgewählt ist, die ein Kniedrehpunkt (13), ein Hüftdrehpunkt (53), einem Knöcheldrehpunkt, einen Ellbogendrehpunkt und einem Schulterdrehpunkt aufweist.

14. Orthese nach einem der Ansprüche 10 bis 13, wobei das Streckkabel (331a) und das Beugekabel (331b) aus einem einzigen Kabel bestehen.

## Revendications

1. Dispositif de répartition de charge (10) destiné à améliorer la mobilité du centre de masse d'un utilisateur lors de mouvements complexes, comprenant :
une ceinture de support pelvien (11) configurée pour être positionnée autour d'une partie inférieure du tronc de l'utilisateur ;
au moins un élément de support de cuisse (12) comprenant deux zones de contact (18) ou plus configurées pour être positionnées selon une configuration agoniste-antagoniste sur une partie postérieure et une partie antérieure d'une cuisse de l'utilisateur, l'au moins un élément de support de cuisse (12) étant relié en rotation à la ceinture de support pelvien (11) ;
au moins un actionneur d'articulation de hanche (22) fournissant un mouvement de rotation de l'au moins un élément de support de cuisse (12) par rapport à la ceinture de support pelvien (11) ;
au moins un élément de support du bas de la jambe (14) comprenant deux zones de contact (18) ou plus configurées pour être positionnées selon une configuration agoniste-antagoniste sur une partie postérieure et une partie antérieure du bas de la jambe de l'utilisateur, l'au moins un élément de support du bas de la jambe (14) étant relié en rotation à l'au moins un élément de support de cuisse (12) ;
au moins un actionneur d'articulation de genou (23) fournissant un mouvement de rotation de l'au moins un élément de support du bas de la jambe (14) par rapport à l'au moins un élément de support de cuisse (12) ;
une pluralité de capteurs (40) positionnés sur la ceinture de support pelvien (11), l'au moins un élément de support de cuisse (12), l'actionneur d'articulation de hanche (22) et l'actionneur d'articulation de genou (23), et au moins un capteur de pied (45) configuré pour être positionné sur un pied de l'utilisateur, la pluralité de capteurs (40, 45) fournissant des signaux mécaniques et biomécaniques ;
une unité de commande (200) reliée fonctionnellement à la pluralité de capteurs (40) et à l'au moins un capteur de pied (45) pour recevoir les signaux mécaniques et biomécaniques, l'unité de commande (200) comportant des instructions exécutables enregistrées dans celle-ci pour traiter et analyser les signaux mécaniques et biomécaniques et générer des points de consigne de mouvement pour des mouvements de l'utilisateur; et
une unité d'alimentation reliée fonctionnellement à l'au moins un actionneur d'articulation de genou (23), à l'au moins un actionneur d'articulation de hanche (22) et à l'unité de commande (200) ; **caractérisé en ce que**
l'au moins un actionneur d'articulation de genou (23) et l'au moins un actionneur d'articulation de hanche (22) transmettent tous deux un mouvement de rotation à une articulation associée de l'utilisateur par l'intermédiaire d'un câble d'extension (331a) et d'un câble de flexion (331b), et transfèrent une contrainte musculosquelettique des articulations vers des segments corporels des membres inférieurs d'un utilisateur, et améliorent ainsi la stabilité des articulations et une amplitude de mouvement des segments corporels, en générant ou en dissipant de l'énergie biomécanique selon des instructions de l'unité de commande (200), en fonction d'un niveau calculé d'énergie correspondant à une réduction de contrainte musculosquelettique au niveau des articulations des membres inférieurs de l'utilisateur nécessaire pour compenser des mouvements de l'utilisateur, l'énergie biomécanique générée ou dissipée étant redistribuée vers la partie inférieure du tronc, la cuisse et le bas de la jambe de l'utilisateur par l'intermédiaire de la ceinture de support pelvien (11), de l'au moins un élément de support de cuisse (12) et de l'au moins un élément de support du bas de la jambe (14), respectivement.

2. Dispositif de répartition de charge (10) selon la revendication 1, comprenant deux éléments de support de cuisse (12), deux éléments de support du bas de la jambe (14), deux actionneurs d'articulation de hanche (22), deux actionneurs d'articulation de genou (23) et deux capteurs de pied (45).

3. Dispositif de répartition de charge (10) selon la revendication 1,
dans lequel l'au moins un actionneur d'articulation de genou (23) et l'au moins un actionneur d'articulation de hanche (22) transfèrent tous deux une contrainte musculosquelettique des articulations vers des segments corporels des membres inférieurs d'un utilisateur, et améliorent ainsi la stabilité des articulations et une amplitude de mouvement des segments corporels, en générant ou en dissipant de l'énergie biomécanique selon des instructions de l'unité de commande (200), en fonction d'un niveau calculé d'énergie correspondant à une réduction de contrainte musculosquelettique au niveau des articulations des membres inférieurs de l'utilisateur nécessaire pour compenser des mouvements de l'utilisateur, l'énergie biomécanique générée ou dissipée étant redistribuée vers la partie inférieure du tronc, la cuisse et le bas de la jambe de l'utilisateur par l'intermédiaire de la ceinture de support pelvien (11), de l'au moins un élément de support de cuisse (12) et de l'au moins un élément de support du bas de la jambe (14), respectivement ;
dans lequel le dispositif de répartition de charge (10) comprend deux éléments de support de cuisse (12), deux éléments de support du bas de la jambe (14), deux actionneurs d'articulation de hanche (22), deux actionneurs d'articulation de genou (23) et deux capteurs de pied (45) ;
dans lequel chacun des éléments de support de cuisse (12) est relié en rotation à au moins un élément de support du bas de la jambe (14) par l'intermédiaire d'un pivot de genou (13) aligné avec un centre de rotation d'une articulation de genou de l'utilisateur et à la ceinture de support pelvien (11) par l'intermédiaire d'un pivot de hanche (53) aligné avec un centre de rotation d'une articulation de hanche de l'utilisateur, et dans lequel au moins l'un des actionneurs d'articulation de genou (23) et des actionneurs d'articulation de hanche (22) est situé à distance du centre de rotation d'une articulation associée de l'utilisateur, l'au moins un des actionneurs d'articulation de genou (23) et des actionneurs d'articulation de hanche (22) transmettant tous deux un mouvement de rotation à l'articulation associée de l'utilisateur par l'intermédiaire d'un câble d'extension (331a) et d'un câble de flexion (331b), le câble d'extension (331a) et le câble de flexion (331b) comprenant chacun un mécanisme de tension.

4. Dispositif de répartition de charge (10) selon la revendication 3, dans lequel chacun des actionneurs d'articulation de genou (23) et chacun des actionneurs d'articulation de hanche (22) est situé à un emplacement sélectionné dans le groupe constitué par un emplacement médial sur une partie latérale respective de la ceinture de support pelvien (11), sur une partie inférieure arrière de la ceinture de support pelvien (11), sur une partie avant respective de la cuisse de l'utilisateur, sur une partie arrière respective de la cuisse de l'utilisateur et sur une partie respective de l'élément de support de cuisse (12) entre une articulation de hanche de l'utilisateur et une articulation de genou de l'utilisateur.

5. Dispositif de répartition de charge (10) selon l'une quelconque des revendications 3 ou 4, dans lequel les éléments de support de cuisse (12) comprennent chacun un mécanisme respectif de réglage de longueur (122).

6. Dispositif de répartition de charge (10) selon la revendication 5, dans lequel le mécanisme de réglage de longueur (122) est sélectionné dans un groupe constitué par un mécanisme coulissant et un mécanisme à vis.

7. Dispositif de répartition de charge (10) selon l'une quelconque des revendications 3 ou 4, comprenant en outre un mécanisme de délocalisation (50) comprenant une liaison structurelle de déport (56) présentant, à une première extrémité, un élément de support d'actionneur (58) configuré pour supporter l'un de l'actionneur d'articulation de genou (23) et de l'actionneur d'articulation de hanche (22), et, à une deuxième extrémité, un élément de liaison au pivot (55) destiné à relier un élément correspondant parmi le pivot de genou (13) et le pivot de hanche (53).

8. Dispositif de répartition de charge (10) selon la revendication 7, dans lequel l'élément de support d'actionneur (58) est configuré pour supporter de manière amovible l'un de l'actionneur d'articulation de genou (23) et de l'actionneur d'articulation de hanche (22).

9. Dispositif de répartition de charge (10) selon l'une quelconque des revendications 3 à 8, dans lequel le câble d'extension (331a) et le câble de flexion (331b) sont formés par un câble unique.

10. Dispositif d'orthèse pour un dispositif de répartition de charge selon la revendication 1, comprenant :
un élément de support proximal comprenant au moins une zone de contact (18) configurée pour être fixée à une partie corporelle proximale d'un utilisateur, et un élément de support distal comprenant au moins une zone de contact (18) configurée pour être fixée à une partie corporelle distale de l'utilisateur, l'élément de support proximal et l'élément de support distal étant reliés en rotation par l'intermédiaire d'un pivot (13, 53) aligné avec un centre de rotation d'une articulation correspondante de l'utilisateur ;
des actionneurs (22, 23) produisant un mouvement de rotation de l'élément de support distal par rapport à l'élément de support proximal, les actionneurs (22, 23) étant situés à distance du centre de rotation de l'articulation correspondante de l'utilisateur, dans lequel les actionneurs (22, 23) comprennent l'au moins un des actionneurs d'articulation de genou (23) et des actionneurs d'articulation de hanche (22), l'au moins un des actionneurs d'articulation de genou (23) et des actionneurs d'articulation de hanche (22) transmettant tous deux un mouvement de rotation au pivot et à l'articulation associée de l'utilisateur par l'intermédiaire d'un câble d'extension (331a) et d'un câble de flexion (331b).

11. Dispositif d'orthèse selon la revendication 10, comprenant en outre un mécanisme de délocalisation (50) comprenant une liaison structurelle de déport (56) présentant, à une première extrémité, un élément de support d'actionneur (58) configuré pour supporter l'un des actionneurs (22, 23), et, à une deuxième extrémité, un élément de liaison au pivot (55) destiné à relier le pivot (13, 53).

12. Dispositif d'orthèse selon la revendication 11, dans lequel l'élément de support d'actionneur (58) est configuré pour supporter de manière amovible l'un des actionneurs (22, 23).

13. Dispositif d'orthèse selon l'une quelconque des revendications 11 ou 12, dans lequel le pivot (13, 53) est sélectionné dans un groupe constitué par un pivot de genou, un pivot de hanche, un pivot de cheville, un pivot de coude et un pivot d'épaule.

14. Dispositif d'orthèse selon l'une quelconque des revendications 10 à 13, dans lequel le câble d'extension (331a) et le câble de flexion (331b) sont formés par un câble unique.
